# EUROPEAN PATENT APPLICATION

(11) **EP 1 002 545 A1**
(43) Date of publication of application: **24.05.2000**
(21) Application number: 99122027.8
(22) Date of filing: 12.11.1999
(51) Int. Cl.: A61K 39/395, A61K 31/415

(54) **Suppressive agent against expression of cell adhesion molecules**

(30) Priority: 13.11.1998 JP 32401398
(71) Applicant: NIPPON ZOKI PHARMACEUTICAL CO., LTD., Chuo-ku, Osaka (JP)
(72) Inventor: Naiki, Mitsuru, Inst. of Bio-Active Science, Yashiro-cho, Katoh-gun, Hyogo (JP)
(74) Representative: HOFFMANN - EITLE

(57) **Abstract**

This invention is directed to a novel suppressive agent against expression of cell adhesion molecules containing histamine-added immunoglobulin as an effective component, having low incidence of side effects. Therefore, the agent of the present invention is very useful as a suppressive agent against expression of cell adhesion having high safety, which can be used for treatment and prevention of various inflammatory diseases.

## Description

### Technical Field

The present invention relates to a novel drug containing histamine-added immunoglobulin as an effective component, more particularly, a suppressive agent against expression of cell adhesion molecules containing histamine-added immunoglobulin as an effective component.

### Prior Art

A complex of immunoglobulin and histamine has been known as a drug preparation, histamine-added immunoglobulin. It restores histamine fixing ability which is lowered in patients suffering from allergy and asthma, and so is used as an agent for nonspecific hyposensitizing therapy for bronchial asthma, allergic rhinitis, vasmotor rhinitis and allergic skin diseases such as urticaria, chronic eczema, atopic dermatitis, etc. Histamine-added immunoglobulin also exhibits a suppressive action against histamine liberation. It does not have any side effects exhibited by antihistamines and adrenocortical hormones used as symptomatic remedies. It has therefore been widely used as a pharmaceutical agent with high safety [See pages 463 and 464 of "Drugs in Japan, Ethical Drugs" (published in October 1996; edited by Japan Pharmaceutical Information Center; published by Yakugyo Jiho Co., Ltd.)].

In addition to said medical indications, it has been reported that histamine-added immunoglobulin has a suppressive action against hypereosinophilicity, immunomodulating action and the like, and the oral administration of histamine-added immunoglobulin expresses the same pharmacological activity as shown by usual hypodermic injection (Japanese Laid-Open Patent Publications Hei-7/53406, Hei-9/31311, etc.). However, in prior articles, there is no description regarding a suppressive action against expression of cell adhesion molecules of histamine-added immunoglobulin.

### Summary of the Invention

The object of the present invention is to provide a novel suppressive agent against expression of cell adhesion molecules, which has high safety with less side effects.

The present inventor has conducted an extensive investigation on said histamine-added immunoglobulin and found that it also has a suppressive action against expression of cell adhesion molecules. The present invention provides a novel suppressive agent against expression of cell adhesion molecules containing histamine-added immunoglobulin as an effective component.

### Brief Explanation of the Drawing:

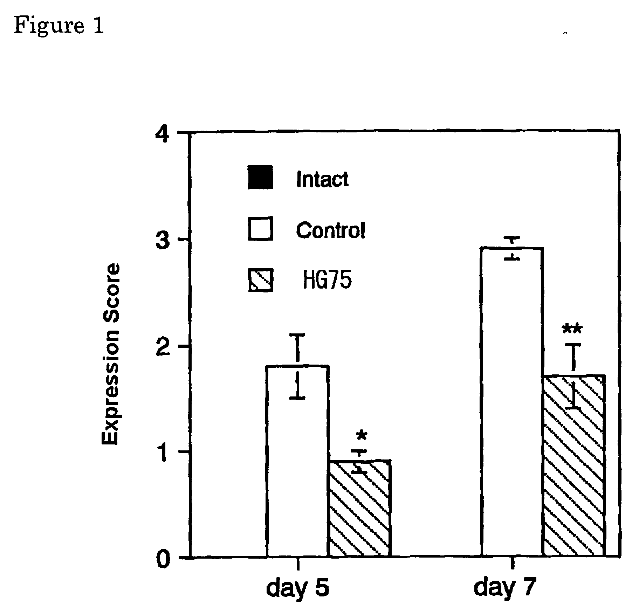

Figure 1 is a graph showing an example of a suppressive action against expression of cell adhesion molecules of the agent of the present invention accompanied with EAE.

### Detailed Description of the Invention

Histamine-added immunoglobulin, which is an effective component of the pharmaceutical agent of the present invention, is a complex of immunoglobulin and histamine, and can be prepared by mixing of an immunoglobulin component with a histamine component to obtain a substantially homogeneous mixture. In the case of an agent used for human being, it goes without saying that human immunoglobulin may be used as a material and such human immunoglobulin can be obtained from serum or placenta plasma by common methods. In order to secure the safety as a pharmaceutical agent, the standards which are usually stipulated for plasma fraction preparations are to be satisfied. For example, human plasma which is negative to HBs antigen, HCV antibody and HIV antibody is used and is further subjected to a heating treatment to avoid the danger of contamination of hepatitis virus and AIDS virus. The heat treatment is commonly used for inactivation of virus and, for example, a liquid-heat treatment at 60°C for 10 hours, a steam-heat treatment at 60°C for 10 hours, a dry-heat treatment at 65°C for 96 hours, etc. are usually conducted for fractionated plasma preparations.

In the case of application to animals other than human being, immunoglobulin may be prepared from an animal other than human being depending upon the species of the animal to be treated. Immunoglobulin has various classes such as IgG, IgA, IgM, etc. As an immunoglobulin component of the present invention, it can be used each of them, and also either solely or jointly together.

Free histamine and its pharmaceutically acceptable salts such as hydrochloride, phosphate and picrate may be used as a histamine component. Histamine dihydrochloride can be employed as a preferable one.

In the manufacture of the pharmaceutical agent of the present invention, it can be prepared, for example, by dissolving 1-200 mg, preferably 5-50 mg, of immunoglobulin and 0.01-2 µg, preferably 0.05-0.5 µg, of histamine component in a suitable solution such as physiological saline, distilled water, etc. with conventional means, mixing and stirring. It can be storable in freezing or as a freeze-drying form.

Histamine-added immunoglobulin of the present invention can be made into pharmaceutical preparations by a combination with a suitable pharmaceutical carriers or diluents. It can be made into various types of preparations by a common method and is able to be made into solid, semisolid, liquid or aerosol formulation for oral or parenteral administration. In preparing the agent, histamine-added immunoglobulin of the present invention can be used either solely or jointly together with other pharmaceutically active components.

In the case of injections, it is preferable to be made into a pharmaceutical composition as an isotonic solution using distilled water for injection or physiological saline solution. In its manufacture, additives such as auxiliary solubilizers, isotonizing agents, stabilizers, buffers, preservatives, etc. may be used in addition to histamine-added immunoglobulin. Examples of the applicable are citric acid, sodium benzoate, glycine, sodium sulfite, sodium bisulfite, sodium pyrosulfite, sodium thiosulfate, cysteine hydrochloride, phosphates, sodium ascorbate, sodium chloride, sodium bicarbonate, etc. Further, the agent of the present invention may be prepared as an injectable preparation which is dissolved upon actual use. Thus, the agent may be prepared in a dry state or a solution filled in vials or the like followed by a freeze-drying. In the manufacture of the dry preparation for injection, fillers such as glucose, mannitol and sorbitol may, if necessary, be added in addition to the above mentioned additives.

In the case of the preparations for oral administration, histamine-added immunoglobulin of the present invention solely or together with commonly used excipients such as a suitable additive (e.g. lactose, mannitol, corn starch, potato starch, etc.) is mixed with binders such as crystalline cellulose, cellulose derivatives, gum arabicum, corn starch, gelatin, etc., disintegrating agents such as corn starch, potato starch, carboxymethylcellulose potassium, etc., lubricating agents such as talc, magnesium stearate, etc. and others including bulking agents, moisturizing agents, buffers, preservatives, perfumes and the like to give tablets, diluted powders, granules or capsules. It is also possible, depending upon the type of the disease or the condition of the patient to prepare the pharmaceutical preparations which are other than those which were mentioned already and are suitable for the therapy such as, for example, inhalations, aerosol preparations, ointments, collyriums, suppositories, etc.

The preferred dose of histamine-added immunoglobulin of the present invention may vary depending upon the type of the disease, the condition of the patient, age or sex of the patient, form of the preparation, method for the administration, term for the administration, etc. and, in order to achieve a desired effect, 1-300 mg, preferably 5-150 mg may be usually given to common adults once or several times a week by hypodermic injection, although the present invention is not particularly limited to such dosage.

Preferred embodiments of the present invention are given as follows.
(1) An suppressive agent against expression of cell adhesion molecules containing histamine-added immunoglobulin as an effective component.
(2) An agent as in (1) wherein the ratio of immunoglobulin to histamine component is 1-200 mg of immunoglobulin component to 0.01-2 µg of histamine component, preferrably 5-50 mg to 0.05-0.5 µg, more preferrably 12.0 mg to 0.15 µg.
(3) An agent according to any of the above (1) to (2), in which immunoglobulin component is human immunoglobulin.
(4) An agent according to any of the above (1) to (3), in which histamine component is histamine dihydrochloride.
(5) An agent according to any of the above (1) to (4) formulated as an injectable preparation.

The following examples, which are illustrative only and not intended to limit the scope of the invention, describes the present invention more concretely.

### Examples

An example of the preparation of histamine-added immunoglobulin of the present invention is shown as follows. Rats were used as experimental animals in the following pharmacological tests and, accordingly, rat immunoglobulin was used in place of human immunoglobulin. Thus, for the pharmacological tests on rats, rat immunoglobulin and histamine dihydrochloride were dissolved in physiological saline solution by the following mixing ratios, stirred at room temperature for 2 hours, freeze-dried and upon use, dissolved by adding a physiological saline solution thereto for hypodermic administration.

| Product of the present invention | Amount of rat immunoglobulin | Amount of histamine.2HCl |
|---|---|---|
| HG 50 | 5,3 mg | 0,10 µg |
| HG 75 | 12,0 mg | 0,15 µg |
| HG 90 | 28,8 mg | 0,30 µg |

Each of HG50, HG75 and HG90 products prepared above exhibited significant effects in all of the following pharmacological tests and, accordingly, only the results obtained for HG75 are given representative thereof.

The pharmacological tests were carried out as follows.

### 1) Animals

Lewis rats (6-10 weeks old, female, SPF) were purchased from Charles River, Japan. Rats were preliminarily maintained for 5 days in an animal room (22 ± 2°C room temperature and 55 ± 10% humidity) with artificial lightning for 12 hours from 8 a.m. to 8 p.m., and then healthy and normal rats were utilized for experiments.

### 2) Preparation of histamine-added rat immunoglobulin and administration thereof

Rat immunoglobulin prepared from normal rat serum (Cohn fractions II and III) and histamine dihydrochloride were dissolved in physiological saline in final concentrations of 26.6 mg/mL and 0.333 µg/mL respectively. The mixture was stirred for 2 hours and then utilized as HG75. HG75 was injected subcutaneously into the back of rat on every second day from immunizing day or cell-transferring day. To a control rat, physiological saline was injected as the same manner as HG75.

### 3) Method for inducing passive experimental allergic encephalomyelitis (pEAE)

The solution (400 µg/mL) of guinia pig myelin basic protein (GB-MBP 68-84) including synthetic peptide (MBP 68-84) corresponding the the encephalitogenic determinants was mixed with an equal volume of complete adjuvant (H37Ra) to give an emulsion. The emulsion (0.1 mL) was subcutaneously administered to the left hind foot pad of Lewis rat anesthetized with diethylether. The spleen cells were isolated from rats 10-14 days after the immunization and the cell suspension was prepared. Erythrocytes were removed by using the solution of 0.75% ammonium chloride and 17 mM TrisHCl (pH = 7.65). After centrifugation, the spleen cells were suspended in RPMI-1640 medium and viable cells were counted by using 0.4% trypan blue solution. The cells were suspended at the concentration of 2 × 10⁶ cells/mL in the medium (RPMI-1640 containing 10 mM HEPES, 20 mM glutamine, 50 µg/mL penicillin, 50 U/mL streptomycin and 5 × 10⁻⁵M 2-mercaptoethanol) supplemented with 7% fetal calf serum. The cells (2 × 10⁷ cells/10 mL) were cultured in a culture dish at 37(C in an atmosphere of 5% CO₂ and 95% air in the presence of 2 µg/mL Concanavalin A. Cultured cells were collected and washed 3 times by centrifugation. The aggregated cells were removed by filtration, the cell concentration was determined by using 0.4% trypan blue. The cell concentration was adjust to 4 × 10⁷ cells/mL by physiological saline. Lewis rat was irradiated with 800 rad X-ray by the X-ray irradiator (MBR-1520R, Hitachi, Japan) and then the cells (2 × 10⁷ cells/0.5 mL) were transferred intravenously into tail vein.

### 4) Clinical Assessment of EAE

The clinical signs of the disease on tested rats were assessed by using clinical index to grade animals on indexes from 0 to 5 as follows. When the clinical sign of grade from 2 to 5 appeared partially, the value by subtracting 0.5 from each grade was adopted. The clinical signs were assessed by a blind method, namely, the treatment of rat such as with or without test drug was not clarified for the operator.

| Grade | Cinical Sign |
|---|---|
| 0 | Normal |
| 0.5 | Slight tail weakness |
| 0.75 | Moderate tail weakness |
| 1 | Disappearance of ability to hold tail up |
| 2 | Inactive |
| 3 | Paralysis of one hind leg |
| 4 | Paralysis of both hind legs |
| 5 | Paralysis of both hind legs accompanied by urinary incontinence |

### 5) Preparation of tissue sample

On the 5th and 7th days after transference of the activated spleen cells, Lewis rats were sacrificed with bleeding and spinal cords were isolated. The segments of lumbar spinal cord were removed and embedded in O.C.T. compound. These segments were frozen by liquid nitrogen and stored at -20°C until use.

### 6) Making and fixing of frozen sections

Cryostat sections (6 µm thickness) of the frozen-stored lumbar spinal cord were prepared and put on gelatin-coated slide glass. The slide samples were air-dried and soaked in acetone at -20°C for 10 minutes. The section samples were air-dried again and stored at -20°C until use.

### 7) Hematoxylin-eosin staining

The slide samples were washed with flowing water for 5 minutes and stained with Meyer's hematoxylin solution for 10minutes. After washing with flowing water for 15 minutes, the samples were stained with eosin solution for 5 minutes. After washing with flowing water to remove excess pigment, the samples were dehydrated with successive, 70%, 90% and 100% ethanol. The samples were fixed with xylene 3 times for 3 minutes and sealed by using entellan.

### 8) Immunohistochemical staining

The slide samples were air-dried and washed with cold phosphate buffered saline (PBS) 3 times for 5 minutes. The samples were soaked in cold PBS containing 0.3% hydrogen peroxide and 0.1% sodium azide for 15 minutes and washed with cold PBS 3 times for 5 minutes. To avoid nonspecific binding of antibody, the samples were incubated with PBS containing 1% bovine serum albumin (blocking buffer) for from 20-30 minutes. After removal of the blocking buffer, the samples were incubated with a solution of biotin-conjugated mouse antibody against rat ICAM-1 and LFA-1 (1/20 diluted with the blocking buffer) for one hour. After washing with cold PBS 2 times for 5 minutes, avidin-biotin-complex prepared by Elite ABC reagent was added and incubated for 30 minutes. After washing with cold PBS 2 times for 5 minutes, the samples were incubated with DAB solution and color reaction was stopped with distilled water by monitoring with microscopy.

### 9) Nuclear staining and sealing method

After the immunohistochemical staining, the slide samples were additionally stained with 1% methyl green solution for 10 minutes. The samples were soaked in 100% ethanol 3 times for one minute and then in xylene 3 times for 5 minutes to dehydrate, and sealed by using entellan.

### 10) Evaluation of stained tissue samples

The immunohistochemical stained samples were monitored with optical microscopy and evaluated the expressions of ICAM-1 and LFA-1 by scoring according to population of expression cells (0: normal, 1: little, 2: weak, 3: moderate, 4: strong, 5: severe).

### 11) Statistical analysis

The results of the evaluations for the immunohistochemical staining was shown as mean ( S.E.M and the significant difference was analyzed with Mann-Whitney u-test (non-parametrical method) or Scheffe's test (covariance method). When the risk (p value) was under 0.05, we decided that this difference was significant.

### 12) Suppressive effect of the pharmaceutical agent of the present invention against expression of cell adhesion molecules on spinal cords with EAE rats

The effects of the agent of this invention on expressions of ICAM-1 which was mainly expressed on vessel endothelial cells and LFA-1 which was a counterpart of ICAM-1 were studied by using immunohistochemical analyses. The example of the results was shown in Figure 1. ICAM-1 expression on intact rats was not observed (score: 0). On the 5th day after cell transfer (early phase), ICAM-1 expression level of control EAE rats was increased (score: 1.8 ± 0.3). On the contrary, the expression level of the treated EAE rats with the agent of this invention was significantly decreased (score: 0.9 ± 0.1) compared to control EAE rats (p < 0.05). On the 7th day after cell transfer (peak phase), ICAM-1 expression level of control EAE rats was more increased (score: 2.9 ± 0.1). It was also observed that the expression level of the treated EAE rats with the present agent was significantly decreased (1.7 ± 0.3) in contrast with control EAE rats (p < 0.01).

Though following results were not shown as a figure, on the 5th day after cell transfer (early phase), LFA-1 expression level of control EAE rats was increased (score: 3.1 ± 0.6) in contrast with intact rats. The expression level of the treated EAE rats with the present agent was significantly decreased (score: 1.0 ± 0.0) compared to control EAE rats (p < 0.05). On the 7th day after cell transfer (peak phase), LFA-1 expression level of control EAE rats was more increased (score: 3.7 ± 0.5). The expression level of the treated EAE rats with the present agent tends to decrease (3.1 ± 0.5) in contrast with control EAE rats.

### Effect of the Invention

Experimental allergic encephalomyelitis (EAE) which has been used as a model for multiple sclerosis has a characteristic of infiltration of immune cells into central nervous system and paralysis of hind legs accompanied by inflammation. The mutual interaction between endothelial cells forming blood-brain barrier and cell adhesion molecules on infiltration cells plays an important role in cell infiltration into central nerve system. ICAM-1 is a representative cell adhesion molecule belonging to immunoglobulin superfamily. It is induced by inflammatory cytokines such as IL-1, TNF and INF-(, and is expressed on endothelial cells and binds to LFA-1 or Mac-1 which is present on leukocytes.

As apparently shown by the results of the above pharmacological tests, the agent of the present invention significantly suppresses the expression of cell adhesion molecules such as ICAM-1 and the like induced by EAE. As mentioned above, it has been clarified that cell adhesion molecules such as ICAM-1 and IFA-1 deeply participate in inflammatory reactions. Therefore, the agent of this invention is useful as a suppressive agent against expression of cell adhesion molecules available for prevention or treatment of inflammatory diseases such as encephalitis, nephritis, myocarditis, vasculitis, enteritis, pneumonia and systemic inflammatory response syndrome (SIRS).

## Claims

1. Use of histamine-added immunoglobulin as an effective component in the production of a pharmaceutical composition effective in suppressing the expression of cell adhesion molecules.

2. Use of histamine-added immunoglobulin as an effective component in the production of a pharmaceutical composition effective in preventing or treating encephalitis, nephritis, myocarditis, vasculitis, enteritis, pneumonia or systemic inflammatory response syndrome (SIRS).

3. Use according to any of claims 1 to 2, wherein the ratio of immunoglobulin to histamine component is 1-200 mg of immunoglobulin component to 0.01-2 µg of histamine component.

4. Use according to claim 3, wherein the ratio is 5-50 mg of immunoglobulin component to 0.05-0.5 µg histamine component.

5. Use according to claim 4, wherein the ratio of immunoglobulin to histamine component is 12.0 mg of immunoglobulin component to 0.15 µg histamine component.

6. Use according to any of claims 1 to 5, wherein the immunoglobulin component is human immunoglobulin.

7. Use according to any of claims 1 to 6, wherein the histamine component is histamine dihydrochloride.

8. Use according to any of claims 1 to 7, wherein the pharmaceutical composition is formulated as an injectable preparation.
